# EUROPEAN PATENT APPLICATION

(11) **EP 4 817 530 A1**
(43) Date of publication of application: **07.10.2026**
(21) Application number: 26169423.6
(22) Date of filing: 31.03.2026
(51) Int. Cl.: A61B 8/08, A61B 8/00, A61B 8/06, G16H 50/30

(54) **ULTRASOUND DIAGNOSTIC APPARATUS AND CONTROL METHOD FOR ULTRASOUND DIAGNOSTIC APPARATUS**

(30) Priority: 02.04.2025 JP 2025061003
(71) Applicant: FUJIFILM Corporation, Tokyo 106-8620 (JP)
(72) Inventor: RAGHAVA, Krishnan, Kaisei (JP)
(74) Representative: Parker, Andrew James

(57) **Abstract**

Provided are an ultrasound diagnostic apparatus and a control method for an ultrasound diagnostic apparatus that can easily and quickly calculate cardiac output.

An ultrasound diagnostic apparatus includes an image classification unit that classifies whether an input image is an ultrasound image obtained by imaging the first cross section or the second cross section, a first measurement unit that measures a cross-sectional area of a left ventricular outflow tract based on the input image in a case where the input image is classified as the ultrasound image obtained by imaging the first cross section, a second measurement unit that measures a velocity-time integral of a left ventricular outflow tract blood flow based on the input image in a case where the input image is classified as the ultrasound image obtained by imaging the second cross section, and a cardiac output calculation unit that calculates cardiac output using the cross-sectional area and the velocity-time integral.

## Description

### BACKGROUND OF THE INVENTION

### 1. Field of the Invention

The present invention relates to an ultrasound diagnostic apparatus that images a heart of a subject and a control method for the ultrasound diagnostic apparatus.

### 2. Description of the Related Art

In the related art, a so-called cardiac output is calculated by capturing an ultrasound image representing a tomographic plane of a heart of a subject using a so-called ultrasound diagnostic apparatus and analyzing the captured ultrasound image. The calculation of the cardiac output is often performed by calculation steps of (1) measuring a diameter of a left ventricular outflow tract in a first ultrasound image representing a so-called parasternal left ventricular long axis view of the heart in a mid-systolic phase, and calculating a cross-sectional area of the left ventricular outflow tract, (2) calculating a velocity-time integral of blood flow in the left ventricular outflow tract using a so-called pulse Doppler method by using a second ultrasound image representing a so-called apical five-chamber view or a so-called apical three-chamber view, (3) calculating a so-called stroke volume by multiplying the cross-sectional area of the left ventricular outflow tract by the velocity-time integral of the blood flow in the left ventricular outflow tract, and (4) calculating the cardiac output by multiplying the stroke volume by a heart rate.

Since the calculation of the cardiac output requires a plurality of procedures in this way, various techniques have been developed to easily measure the stroke volume and the cardiac output. For example, JP2024-129281A discloses a technique of causing to a trained model in so-called machine learning to automatically output a velocity-time integral of blood flow by inputting a Doppler waveform image to the trained model.

### SUMMARY OF THE INVENTION

However, even in a case where the technique disclosed in JP2024-129281A is used, the user still needs to perform a plurality of procedures, and a large amount of time is required to calculate the cardiac output.

The present invention has been made in order to solve such a problem in the related art, and an object of the present invention is to provide an ultrasound diagnostic apparatus and a control method for an ultrasound diagnostic apparatus capable of easily and quickly calculating cardiac output.

The above-described object can be achieved with the following configurations.
[1] An ultrasound diagnostic apparatus that measures cardiac output based on ultrasound images of a plurality of frames obtained by respectively imaging a first cross section and a second cross section, which are different from each other, of a heart of a subject, the ultrasound diagnostic apparatus comprising:
   an image classification unit that classifies whether an input image is an ultrasound image obtained by imaging the first cross section or the second cross section;
   a first measurement unit that measures a cross-sectional area of a left ventricular outflow tract based on the input image in a case where the image classification unit classifies the input image as the ultrasound image obtained by imaging the first cross section;
   a second measurement unit that measures a velocity-time integral of a left ventricular outflow tract blood flow based on the input image in a case where the image classification unit classifies the input image as the ultrasound image obtained by imaging the second cross section; and
   a cardiac output calculation unit that calculates the cardiac output by using the cross-sectional area measured by the first measurement unit and the velocity-time integral measured by the second measurement unit.
[2] The ultrasound diagnostic apparatus according to [1],
   in which the image classification unit classifies the input image using a parasternal left ventricular long axis view as the first cross section and an apical five-chamber view or an apical three-chamber view as the second cross section.
[3] The ultrasound diagnostic apparatus according to [2],
   in which the first measurement unit includes
   a left ventricular outflow tract diameter measurement unit that measures a left ventricular outflow tract diameter based on the input image,
   a mid-systolic frame detection unit that detects a mid-systolic frame based on the input image, and
   a cross-sectional area calculation unit that calculates the cross-sectional area of the left ventricular outflow tract from the left ventricular outflow tract diameter measured by the left ventricular outflow tract diameter measurement unit in the mid-systolic frame detected by the mid-systolic frame detection unit.
[4] The ultrasound diagnostic apparatus according to [3],
   in which the left ventricular outflow tract diameter measurement unit measures the left ventricular outflow tract diameter at a position within a predetermined distance from an insertion point of an aortic valve toward a heart side.
[5] The ultrasound diagnostic apparatus according to [3] or [4],
   in which the mid-systolic frame detection unit detects a frame in which an angle between a pair of aortic valve leaflets is maximized as the mid-systolic frame.
[6] The ultrasound diagnostic apparatus according to any one of [3] to [5],
   in which the first measurement unit includes a frame reliability degree calculation unit that calculates a reliability degree of the frame, and
   the left ventricular outflow tract diameter measurement unit measures the left ventricular outflow tract diameter based on the frame in which the reliability degree calculated by the frame reliability degree calculation unit is equal to or greater than a predetermined reliability degree threshold value.
[7] The ultrasound diagnostic apparatus according to [6],
   in which the frame reliability degree calculation unit calculates the reliability degree based on a certainty of classification in the image classification unit or based on a time-series change in the left ventricular outflow tract diameter measured by the left ventricular outflow tract diameter measurement unit.
[8] The ultrasound diagnostic apparatus according to [2],
   in which the second measurement unit includes
   a systole recognition unit that recognizes a systole based on the input image,
   a gate setting unit that sets a Doppler gate in the input image recognized as the systole by the systole recognition unit,
   a Doppler waveform acquisition unit that acquires a Doppler waveform in the Doppler gate set by the gate setting unit, and
   a velocity-time integral calculation unit that calculates the velocity-time integral based on the Doppler waveform acquired by the Doppler waveform acquisition unit.
[9] The ultrasound diagnostic apparatus according to [8],
   in which the systole recognition unit recognizes the systole based on any one of closure of a mitral valve, presence or absence of depiction of an aortic valve, an area of a left ventricle, or a depth of a reference point set in the input image.
[10] The ultrasound diagnostic apparatus according to [8] or [9],
   in which the first measurement unit includes a left ventricular outflow tract diameter measurement unit that measures a left ventricular outflow tract diameter based on the input image, and
   the gate setting unit sets the Doppler gate in the input image based on the left ventricular outflow tract diameter measured by the left ventricular outflow tract diameter measurement unit in the first measurement unit.
[11] The ultrasound diagnostic apparatus according to [3],
   in which the cardiac output calculation unit
   calculates a heart rate based on the number of plots of the left ventricular outflow tract diameter by the left ventricular outflow tract diameter measurement unit between two consecutive mid-systolic frames detected by the mid-systolic frame detection unit, and
   calculates the cardiac output from the cross-sectional area measured by the first measurement unit, the velocity-time integral measured by the second measurement unit, and the calculated heart rate.
[12] The ultrasound diagnostic apparatus according to [3], further comprising:
   an error detection unit that detects an error in the left ventricular outflow tract diameter measured by the left ventricular outflow tract diameter measurement unit.
[13] The ultrasound diagnostic apparatus according to [12],
   in which the error detection unit detects the error in the left ventricular outflow tract diameter by comparing a time-series change curve of the left ventricular outflow tract diameter measured by the left ventricular outflow tract diameter measurement unit with a predetermined curve or by comparing the left ventricular outflow tract diameter measured by the left ventricular outflow tract diameter measurement unit with a predetermined set value.
[14] The ultrasound diagnostic apparatus according to [13], further comprising:
   a correction unit that corrects the left ventricular outflow tract diameter in which the error is detected by the error detection unit based on the cross-sectional area of the left ventricular outflow tract calculated by the cross-sectional area calculation unit in the past.
[15] The ultrasound diagnostic apparatus according to [8], further comprising:
   an error detection unit that detects an error in the velocity-time integral calculated by the velocity-time integral calculation unit.
[16] The ultrasound diagnostic apparatus according to [15],
   in which the error detection unit detects the error in the velocity-time integral by comparing a Doppler curve obtained by tracing the Doppler waveform acquired by the Doppler waveform acquisition unit with a predetermined curve or by comparing the velocity-time integral calculated by the velocity-time integral calculation unit with a predetermined set value.
[17] The ultrasound diagnostic apparatus according to [16], further comprising:
   a correction unit that corrects the velocity-time integral in which the error is detected by the error detection unit based on the velocity-time integral calculated by the velocity-time integral calculation unit in the past.
[18] A control method for an ultrasound diagnostic apparatus that measures cardiac output based on an ultrasound image of a plurality of frames obtained by respectively imaging a first cross section and a second cross section, which are different from each other, of a heart of a subject, the control method comprising:
   classifying whether an input image is an ultrasound image obtained by imaging the first cross section or the second cross section;
   measuring a cross-sectional area of a left ventricular outflow tract based on the input image classified as the ultrasound image obtained by imaging the first cross section;
   measuring a velocity-time integral of a left ventricular outflow tract blood flow based on the input image classified as the ultrasound image obtained by imaging the second cross section; and
   calculating the cardiac output using the measured cross-sectional area and the measured velocity-time integral.

In the present invention, the ultrasound diagnostic apparatus includes the image classification unit that classifies whether the input image is the ultrasound image obtained by imaging any of the first cross section or the second cross section, the first measurement unit that measures the cross-sectional area of the left ventricular outflow tract based on the input image in a case where the image classification unit classifies the input image as the ultrasound image obtained by imaging the first cross section, the second measurement unit that measures the velocity-time integral of the left ventricular outflow tract blood flow based on the input image in a case where the image classification unit classifies the input image as the ultrasound image obtained by imaging the second cross section, and the cardiac output calculation unit that calculates the cardiac output using the cross-sectional area measured by the first measurement unit and the velocity-time integral measured by the second measurement unit, and thus the cardiac output can be easily and quickly calculated.

### BRIEF DESCRIPTION OF THE DRAWINGS

FIG. 1 is a block diagram showing a configuration of an ultrasound diagnostic apparatus according to Embodiment 1 of the present invention.
FIG. 2 is a block diagram showing an internal configuration of a transmission and reception circuit in Embodiment 1 of the present invention.
FIG. 3 is a block diagram showing an internal configuration of an image generation unit in Embodiment 1 of the present invention.
FIG. 4 is a diagram showing an example of an ultrasound image representing a parasternal left ventricular long axis view.
FIG. 5 is a diagram showing an example of an ultrasound image representing an apical five-chamber view.
FIG. 6 is a block diagram showing an internal configuration of a first measurement unit in Embodiment 1 of the present invention.
FIG. 7 is a diagram showing an example of a measurement line set in the ultrasound image representing the parasternal left ventricular long axis view.
FIG. 8 is a block diagram showing an internal configuration of a second measurement unit in Embodiment 1 of the present invention.
FIG. 9 is a diagram showing an example of a Doppler gate set in the ultrasound image representing the apical five-chamber view.
FIG. 10 is a flowchart showing an operation of the ultrasound diagnostic apparatus according to Embodiment 1 of the present invention.
FIG. 11 is a diagram showing an example of a series of processing steps displayed on a monitor.
FIG. 12 is a block diagram showing an internal configuration of a first measurement unit in a modification example of Embodiment 1 of the present invention.
FIG. 13 is a block diagram showing a configuration of an ultrasound diagnostic apparatus according to Embodiment 2 of the present invention.
FIG. 14 is a diagram showing an example of a time-series change curve of a left ventricular outflow tract diameter and a predetermined curve compared with the time-series change curve.

### DESCRIPTION OF THE PREFERRED EMBODIMENTS

Hereinafter, an embodiment of the present invention will be described with reference to the accompanying drawings.

Constituent elements will be described below based on a representative embodiment of the present invention, but the present invention is not limited to such an embodiment.

In the present specification, numerical ranges represented by "to" include numerical values before and after "to" as lower limit values and upper limit values.

In the present specification, "identical" and "the same" include an error range generally allowed in the technical field.

### Embodiment 1

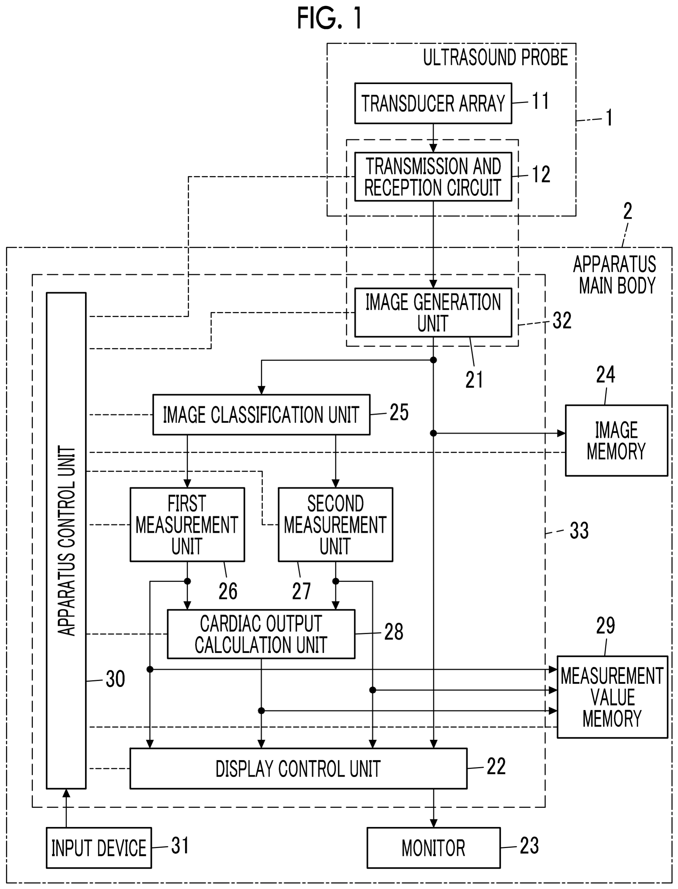

FIG. 1 shows a configuration of an ultrasound diagnostic apparatus according to Embodiment 1 of the present invention. The ultrasound diagnostic apparatus comprises an ultrasound probe 1 and an apparatus main body 2 connected to each other by so-called wired communication or so-called wireless communication.

The ultrasound probe 1 comprises a transducer array 11 and a transmission and reception circuit 12 connected to the transducer array 11.

The apparatus main body 2 comprises an image generation unit 21 connected to the transmission and reception circuit 12. In the apparatus main body 2, a display control unit 22 and a monitor 23 are sequentially connected to the image generation unit 21. In addition, an image memory 24 and an image classification unit 25 are connected to the image generation unit 21. A first measurement unit 26 and a second measurement unit 27 are connected to the image classification unit 25. A cardiac output calculation unit 28 is connected to the first measurement unit 26 and the second measurement unit 27. A measurement value memory 29 and the display control unit 22 are connected to the first measurement unit 26, the second measurement unit 27, and the cardiac output calculation unit 28. In addition, an apparatus control unit 30 is connected to the transmission and reception circuit 12, the image generation unit 21, the display control unit 22, the image memory 24, the image classification unit 25, the first measurement unit 26, the second measurement unit 27, the cardiac output calculation unit 28, and the measurement value memory 29. An input device 31 is connected to the apparatus control unit 30.

The transmission and reception circuit 12 and the image generation unit 21 constitute an image acquisition unit 32. In addition, a processor 33 for the apparatus main body 2 is configured by the image generation unit 21, the display control unit 22, the image classification unit 25, the first measurement unit 26, the second measurement unit 27, and the cardiac output calculation unit 28.

The transducer array 11 of the ultrasound probe 1 has a plurality of ultrasound transducers that are one-dimensionally or two-dimensionally arranged. In accordance with a drive signal supplied from the transmission and reception circuit 12, each of the ultrasound transducers transmits an ultrasound wave and receives an ultrasound echo from a subject to output a signal based on the ultrasound echo. Each ultrasound transducer is configured by forming electrodes at both ends of a piezoelectric body consisting of, for example, a piezoelectric ceramic represented by lead zirconate titanate (PZT), a polymer piezoelectric element represented by polyvinylidene difluoride (PVDF), and a piezoelectric single crystal represented by a lead magnesium niobate-lead titanate (PMN-PT).

The image acquisition unit 32 configured by the transmission and reception circuit 12 and the image generation unit 21 acquires ultrasound images of a plurality of frames as a moving image in which a heart of the subject is imaged, by transmitting and receiving ultrasound beams using the ultrasound probe 1.

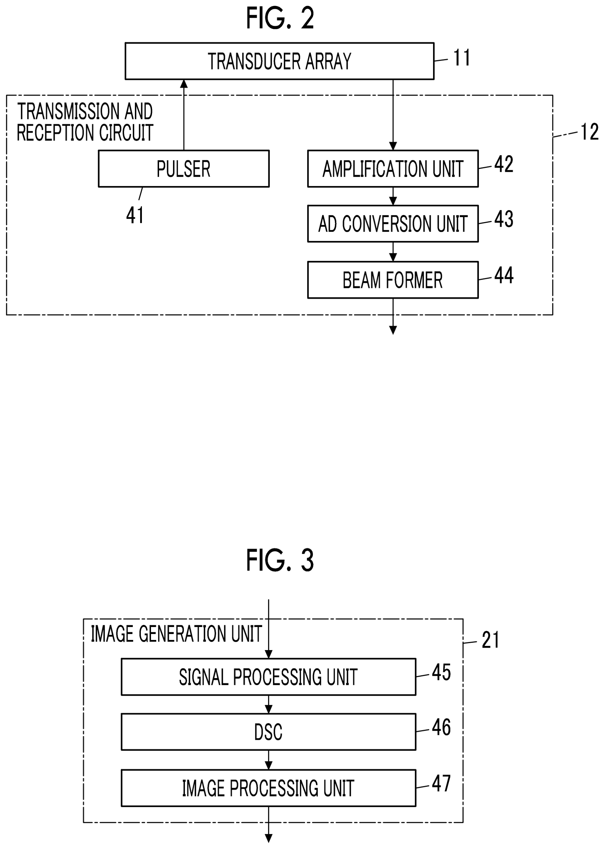

The transmission and reception circuit 12 transmits the ultrasound waves from the transducer array 11 and generates a sound ray signal based on reception signals acquired by the transducer array 11 under control of the apparatus control unit 30. As shown in FIG. 2, the transmission and reception circuit 12 includes a pulser 41 connected to the transducer array 11, and an amplification unit 42, an analog-to-digital (AD) conversion unit 43, and a beam former 44 that are sequentially connected in series to the transducer array 11.

The pulser 41 includes, for example, a plurality of pulse generators, adjusts a delay amount of each drive signal based on a transmission delay pattern selected in accordance with a control signal from the apparatus control unit 30 so that the ultrasound waves transmitted from the plurality of ultrasound transducers of the transducer array 11 form an ultrasound beam, and supplies each drive signal to the plurality of ultrasound transducers. In this way, in a case where a pulsed or continuous wave-like voltage is applied to the electrodes of the ultrasound transducer of the transducer array 11, the piezoelectric body expands and contracts to generate a pulsed or continuous wave-like ultrasound wave from each of the ultrasound transducers, thereby forming an ultrasound beam from the combined wave of these ultrasound waves.

The transmitted ultrasound beam is reflected by a target, for example, a site of the subject, and propagates toward the transducer array 11 of the ultrasound probe 1. The ultrasound echo propagating toward the transducer array 11 is received by each ultrasound transducer constituting the transducer array 11. In this case, each of the ultrasound transducers constituting the transducer array 11 receives the propagating ultrasound echo to expand and contract to generate the reception signal, which is an electrical signal, and outputs these reception signals to the amplification unit 42.

The amplification unit 42 amplifies the signal input from each of the ultrasound transducers constituting the transducer array 11 and transmits the amplified signal to the AD conversion unit 43. The AD conversion unit 43 converts the signal transmitted from the amplification unit 42 into digital reception data. The beam former 44 performs so-called reception focus processing by applying and adding the delay to each reception data received from the AD conversion unit 43. By the reception focus processing, each reception data converted by the AD conversion unit 43 is phase-added, and the sound ray signal in which the focus of the ultrasound echo is narrowed down is acquired.

As shown in FIG. 3, the image generation unit 21 has a configuration in which a signal processing unit 45, a digital scan converter (DSC) 46, and an image processing unit 47 are sequentially connected in series to each other.

The signal processing unit 45 corrects attenuation by distance of the sound ray signal received from the transmission and reception circuit 12 in accordance with depths of reflection positions of the ultrasound waves using a sound speed value set by the apparatus control unit 30 and then performs envelope detection processing on the sound ray signal to generate a B-mode image signal that is tomographic image information related to tissues inside the subject.

The DSC 46 converts (raster-converts) the B-mode image signal generated by the signal processing unit 45 into an image signal in accordance with a normal television signal scanning method.

The image processing unit 47 performs various types of necessary image processing such as gradation processing on the B-mode image signal input from the DSC 46, and then sends the B-mode image signal to the display control unit 22, the image memory 24, and the image classification unit 25. Hereinafter, the B-mode image signal that is image-processed by the image processing unit 47 will be referred to as an ultrasound image.

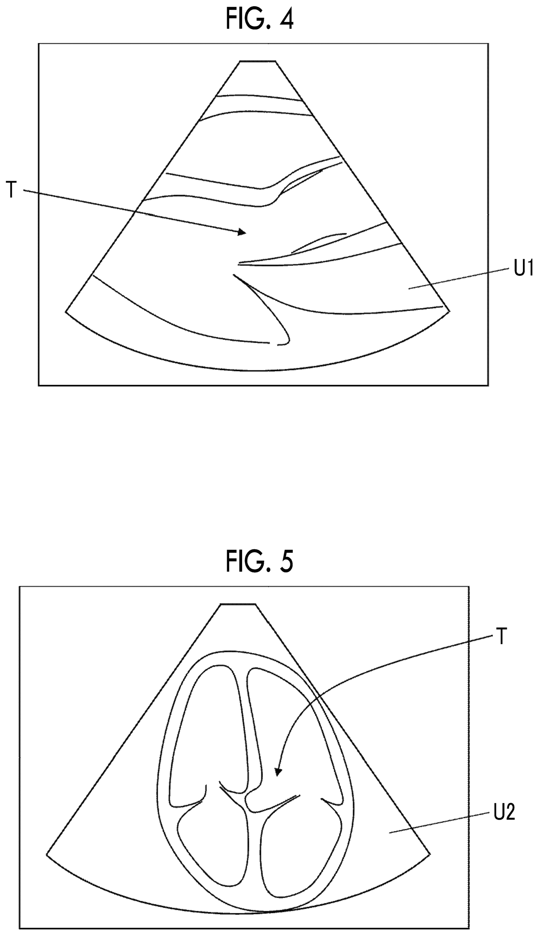

In the present invention, the image acquisition unit 32 acquires ultrasound images of a plurality of frames in which a first cross section and a second cross section different from each other of a heart of a subject are respectively imaged. For example, as schematically shown in FIG. 4, the image acquisition unit 32 can acquire an ultrasound image representing a so-called parasternal left ventricular long axis view as an ultrasound image U1 representing the first cross section. The parasternal left ventricular long axis view is a cross section including a left ventricle, a left ventricular outflow tract T, a left atrium, and a right ventricle. In addition, for example, as schematically shown in FIG. 5, the image acquisition unit 32 can acquire an ultrasound image representing a so-called apical five-chamber view as an ultrasound image U2 representing the second cross section. The apical five-chamber view is a cross section of the heart including five lumens of the left ventricle, the left ventricular outflow tract T, the left atrium, the right ventricle, and the right atrium. Although not shown, the image acquisition unit 32 can also acquire an ultrasound image representing a so-called apical three-chamber view instead of the ultrasound image U2 representing the apical five-chamber view. The apical three-chamber view is a cross section of the heart including three lumens of the left ventricle, the left atrium, and the right ventricle. Both the apical five-chamber view and the apical three-chamber view include the left ventricular outflow tract T.

The image memory 24 is a memory that stores the ultrasound images of the plurality of frames acquired by the image acquisition unit 32. The user can use the image memory 24, for example, to check the ultrasound images of the plurality of frames stored in the image memory 24 after the examination. Here, as the image memory 24, for example, a recording medium such as a flash memory, a hard disk drive (HDD), a solid-state drive (SSD), a flexible disk (FD), a magneto-optical disk (MO disk), a magnetic tape (MT), a random-access memory (RAM), a compact disc (CD), a digital versatile disc (DVD), a secure digital card (SD card), or a universal serial bus memory (USB memory) can be used.

The image classification unit 25 classifies whether the input image among the ultrasound images of the plurality of frames acquired by the image acquisition unit 32 is an ultrasound image obtained by imaging the first cross section or the second cross section. The image classification unit 25 can classify the ultrasound image by, for example, storing template data representing typical image patterns of the parasternal left ventricular long axis view, the apical five-chamber view, and the apical three-chamber view in advance and recognizing any of the parasternal left ventricular long axis view, the apical five-chamber view, and the apical three-chamber view using a so-called template matching method of searching for the ultrasound image using the template data. The image classification unit 25 can also have a trained model in so-called machine learning in which a large number of ultrasound images representing the parasternal left ventricular long axis view, the apical five-chamber view, and the apical three-chamber view are to be learned, and can cause the trained model to output a classification result by inputting the ultrasound image to the trained model. The image classification unit 25 can also use an algorithm such as scale-invariant feature transform (SIFT) or support vector machine (SVM).

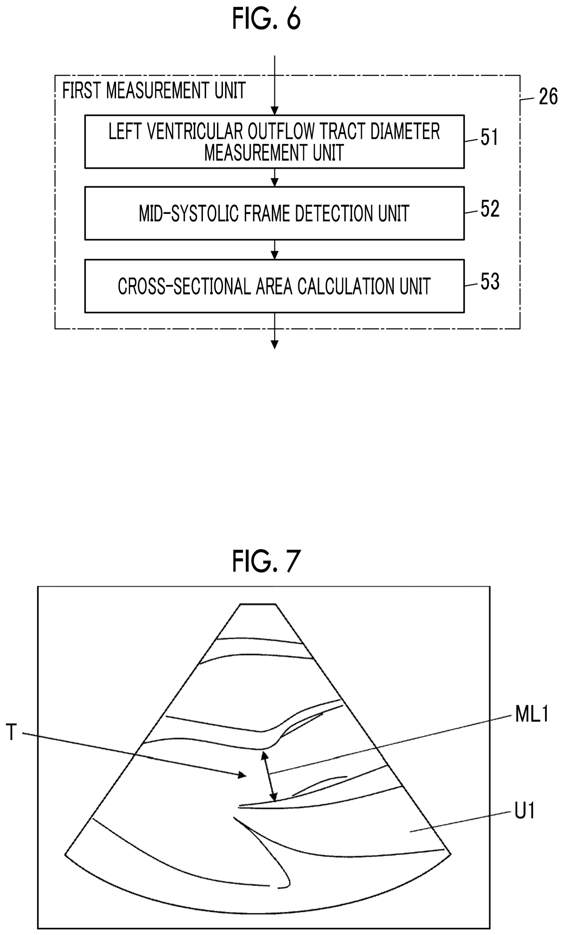

The first measurement unit 26 measures a cross-sectional area (CSA) of the left ventricular outflow tract T based on the classified input image in a case where the image classification unit 25 classifies the input image as the ultrasound image U1 obtained by imaging the first cross section. As shown in FIG. 6, the first measurement unit 26 has a configuration in which a left ventricular outflow tract diameter measurement unit 51, a mid-systolic frame detection unit 52, and a cross-sectional area calculation unit 53 are connected in series.

The left ventricular outflow tract diameter measurement unit 51 measures a diameter of the left ventricular outflow tract T, that is, the left ventricular outflow tract diameter based on the input image. The left ventricular outflow tract diameter measurement unit 51 detects the aortic valve connected to the left ventricular outflow tract T by analyzing the input image, and disposes a measurement line ML1 as shown in FIG. 7 at a position within a predetermined distance from the detected aortic valve toward the heart side, more specifically, toward the left ventricle, for example, within 1 cm to 2 cm. The left ventricular outflow tract diameter measurement unit 51 measures a length of the disposed measurement line ML1 as the left ventricular outflow tract diameter. The left ventricular outflow tract diameter measurement unit 51 can detect the aortic valve from the input image using a known algorithm such as a template matching method or a method using machine learning.

The mid-systolic frame detection unit 52 detects a mid-systolic frame representing a mid-systolic phase of the heart based on the input image. In a guideline issued by the American Society of Echocardiography (ASE) or the like, a so-called phase in which the aortic valve is most open is defined as the mid-systolic phase of the heart. Therefore, the mid-systolic frame detection unit 52 can also detect, for example, a frame in which an angle between a pair of aortic valve leaflets is maximized as the mid-systolic frame. In this case, the mid-systolic frame detection unit 52 can detect the aortic valve from the ultrasound images of the plurality of frames using a known algorithm such as a template matching method or a method using machine learning.

The cross-sectional area calculation unit 53 calculates a cross-sectional area of the left ventricular outflow tract T from the left ventricular outflow tract diameter measured by the left ventricular outflow tract diameter measurement unit 51 in the mid-systolic frame detected by the mid-systolic frame detection unit 52. In this case, the cross-sectional area calculation unit 53 can calculate the cross-sectional area of the left ventricular outflow tract T by, for example, regarding the cross-sectional shape of the left ventricular outflow tract T as a circle.

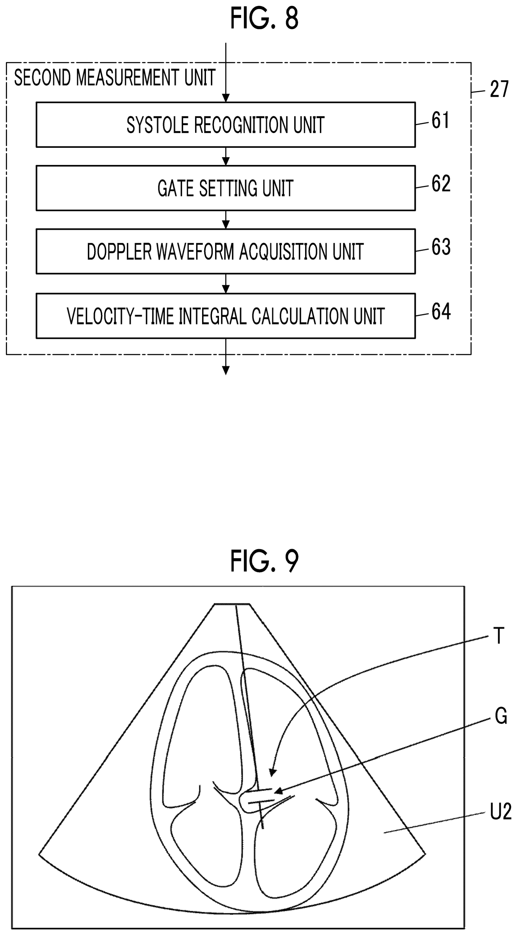

In a case where the image classification unit 25 classifies the input image as the ultrasound image U2 obtained by imaging the second cross section, the second measurement unit 27 measures a velocity-time integral of the blood flow in the left ventricular outflow tract T, that is, the left ventricular outflow tract blood flow based on the input image. As shown in FIG. 8, the second measurement unit 27 has a configuration in which a systole recognition unit 61, a gate setting unit 62, a Doppler waveform acquisition unit 63, and a velocity-time integral calculation unit 64 are connected in series.

The systole recognition unit 61 recognizes the systole of the heart based on the input image. Here, in the systole of the heart, a so-called mitral valve is closed to prevent the backflow of blood to the left atrium. Therefore, the systole recognition unit 61 can recognize, for example, a period corresponding to a frame in which the mitral valve is closed in the ultrasound images U2 of the plurality of frames as the systole of the heart by analyzing the ultrasound images U2 of the plurality of frames. In this case, the systole recognition unit 61 can detect the mitral valve from the ultrasound images U2 of the plurality of frames by using, for example, a known algorithm such as a template matching method or a method using machine learning.

In addition, it is known that the aortic valve is opened in the systole of the heart, but the opened aortic valve is usually difficult to be depicted in the ultrasound image U2 representing the second cross section. Therefore, the systole recognition unit 61 can recognize, for example, a period corresponding to the ultrasound image U2 of a frame in which the aortic valve is not detected as the systole of the heart by performing processing of detecting the aortic valve from the ultrasound images U2 of the plurality of frames. In this case, the systole recognition unit 61 can perform processing of detecting the aortic valve from the ultrasound images U2 of the plurality of frames by using, for example, a known algorithm such as a template matching method or a method using machine learning.

In addition, it is known that the volume of the left ventricle is reduced in the systole of the heart. Therefore, the systole recognition unit 61 measures the area of the left ventricle in the ultrasound images U2 of the plurality of frames, and can recognize, for example, a period in which the area of the left ventricle is reduced in the time series as the systole of the heart. In this case, the systole recognition unit 61 can detect the left ventricle from the ultrasound images U2 of the plurality of frames by using, for example, a known algorithm such as a template matching method or a method using machine learning. In addition, the systole recognition unit 61 can calculate the area of the left ventricle, for example, by measuring the number of pixels of the image region of the left ventricle detected in the ultrasound image U2.

In addition, the position of the aorta fluctuates with the heartbeat, so that the depth of the anatomical structure in a certain range including the aorta fluctuates in the ultrasound images U2 of the plurality of frames. Since the anatomical structure in a certain range including the aorta changes to be deeper in the systole of the heart in the ultrasound images U2 of the plurality of frames, the systole recognition unit 61 sets at least one reference point in the vicinity of the aorta in the ultrasound images U2 of the plurality of frames, and can recognize, for example, a period in which at least one set reference point becomes deeper as the systole of the heart. In this case, the systole recognition unit 61 can set, for example, at least one reference point at a so-called aortic valve root or the center of the aorta.

As described above, the systole recognition unit 61 can recognize the systole of the heart based on any one of the closure of the mitral valve, the presence or absence of depiction of the aortic valve, the area of the left ventricle, or the depth of at least one reference point set in the input image.

As shown in FIG. 9, the gate setting unit 62 sets the Doppler gate G in the input image, which is the ultrasound image U2 of the plurality of frames recognized as the systole by the systole recognition unit 61. The gate setting unit 62 can detect the left ventricular outflow tract T from the input image by using, for example, a known algorithm such as a template matching method or a method using machine learning, and can dispose the Doppler gate G in the detected left ventricular outflow tract T.

In addition, the gate setting unit 62 can also set the Doppler gate G in the input image based on the left ventricular outflow tract diameter measured by the left ventricular outflow tract diameter measurement unit 51 in the first measurement unit 26. In this case, the gate setting unit 62 can dispose, for example, the measurement line ML1 at a plurality of positions of the left ventricular outflow tract T in the ultrasound image U2, and can dispose the Doppler gate G at a position of the measurement line ML1 from which the measurement value closest to the left ventricular outflow tract diameter measured by the left ventricular outflow tract diameter measurement unit 51 is obtained.

The Doppler waveform acquisition unit 63 acquires the Doppler waveform in the Doppler gate G set by the gate setting unit 62. In this case, the Doppler waveform acquisition unit 63 mixes the sound ray signal received from the transmission and reception circuit 12 with a carrier signal having a reference frequency to quadrature-detect the sound ray signal and convert the sound ray signal into a complex signal, and removes a frequency component derived from the movement of the in vivo tissue of the subject from the complex signal. The Doppler waveform acquisition unit 63 further performs frequency analysis by performing Fourier transformation on the complex signals of the plurality of sample points to obtain the blood flow velocity, generates a spectrum signal, and acquires a Doppler waveform image signal representing the Doppler waveform by representing the magnitude of each frequency component with brightness while aligning the spectrum signal on the time axis. The Doppler waveform image signal is called a so-called Doppler image, represents a time-varying waveform of the blood flow velocity, shows a time axis on a horizontal axis, shows a Doppler shift frequency, that is, a flow velocity on a vertical axis, and shows power at each frequency component with brightness of the waveform.

The velocity-time integral calculation unit 64 calculates a velocity-time integral (VTI) based on the Doppler waveform acquired by the Doppler waveform acquisition unit 63. The velocity-time integral calculation unit 64 specifies a cardiac cycle period in the Doppler waveform, and calculates the velocity-time integral by performing time integration of the value of the flow velocity in the Doppler waveform over the cardiac cycle period. The velocity-time integral calculation unit 64 can specify a repeating unit of the waveform in the Doppler waveform by, for example, analyzing the Doppler waveform, and can specify a period to which the specified repeating unit belongs as the cardiac cycle period.

The processing of calculating the velocity-time integral by the Doppler waveform acquisition unit 63 and the velocity-time integral calculation unit 64 is performed after the drive signal is sent to the transducer array 11 to transmit the ultrasonic waves in a pulsed manner under the control of the transmission and reception circuit 12 and the apparatus control unit 30.

The cardiac output calculation unit 28 calculates the cardiac output using the cross-sectional area of the left ventricular outflow tract T measured by the first measurement unit 26 and the velocity-time integral of the left ventricular outflow tract blood flow measured by the second measurement unit 27. More specifically, the cardiac output calculation unit 28 calculates a so-called stroke volume by multiplying the cross-sectional area of the left ventricular outflow tract T by the velocity-time integral, and calculates the cardiac output by multiplying the calculated stroke volume by the heart rate. The cardiac output calculation unit 28 can calculate, for example, a reciprocal of a period of the specified repeating waveform as the heart rate by specifying a repeating waveform in the time-varying waveform of the blood flow velocity acquired by the Doppler waveform acquisition unit 63 of the second measurement unit 27.

In addition, the cardiac output calculation unit 28 can also calculate the heart rate based on the number of plots of the left ventricular outflow tract diameter by the left ventricular outflow tract diameter measurement unit 51 between two consecutive mid-systolic frames detected by the mid-systolic frame detection unit 52 of the first measurement unit 26.

The measurement value memory 29 is a memory that stores the left ventricular outflow tract diameter and the cross-sectional area of the left ventricular outflow tract T measured by the first measurement unit 26, the Doppler waveform and the velocity-time integral of the left ventricular outflow tract blood flow measured by the second measurement unit 27, and the stroke volume and the cardiac output calculated by the cardiac output calculation unit 28. As the measurement value memory 29, for example, a recording medium such as a flash memory, an HDD, an SSD, an FD, an MO disk, an MT, a RAM, a CD, a DVD, an SD card, or a USB memory can be used.

In a case where the user disposes the ultrasound probe 1 on the body surface of the subject to capture the ultrasound images U1 and U2, the apparatus control unit 30 controls each unit such that the processing in the image classification unit 25, the first measurement unit 26, the second measurement unit 27, and the cardiac output calculation unit 28 is sequentially and automatically performed. As described above, the user can automatically obtain the measurement value of the cardiac output by simply performing the operation of disposing the ultrasound probe 1 on the body surface of the subject. Therefore, the user does not need to perform a complicated operation to measure the cardiac output, and can easily and quickly measure the cardiac output.

The display control unit 22 performs predetermined processing on the ultrasound images U1 and U2 acquired by the image acquisition unit 32, the measurement line ML1 and the obtained measurement value set by the first measurement unit 26, the Doppler gate G and the obtained measurement value set by the second measurement unit 27, the measurement value of the cardiac output obtained by the cardiac output calculation unit 28, and the like, and displays the ultrasound images U1 and U2, the measurement line ML1, the obtained measurement value, the Doppler gate G, the obtained measurement value, the measurement value of the cardiac output, and the like on the monitor 23 under the control of the apparatus control unit 30.

The monitor 23 displays the ultrasound images U1 and U2, the measurement line ML1, the Doppler gate G, the cross-sectional area of the left ventricular outflow tract T, the velocity-time integral, the measurement value of the cardiac output, and the like under the control of the display control unit 22, and has, for example, a display device such as a liquid crystal display (LCD) or an organic electroluminescence display (organic EL display).

The input device 31 is an input device for the user to perform an input operation, and is configured by, for example, a device such as a keyboard, a mouse, a trackball, a touchpad, and a touch sensor disposed in a state of being superimposed on the monitor 23.

In the present embodiment, each process in the processor 33 is executed by any computer. In addition, any computer may execute these processes using a processor as hardware, a program as software, or a combination thereof. In such a case, the processor is configured to execute various types of processes in the present embodiment in cooperation with the program, and may function as each unit or each means in the present embodiment. Further, the execution order of the processes by the processor is not limited to the order described and may be changed as appropriate. Any computer may be a general purpose computer, a special purpose computer, a workstation, or other system capable of executing each process.

The processor 33 may be configured by one or a plurality of pieces of hardware, and the type of hardware is not limited. For example, the processor 33 may be configured by hardware such as a central processing unit (CPU), a micro processing unit (MPU), a programmable logic device such as a field programmable gate array (FPGA), a dedicated circuit for executing specific processing, such as an application specific integrated circuit (ASIC), a graphics processing unit (GPU), or a neural processing unit (NPU). Additionally, the type of hardware may be a combination of different types of hardware. In a case where a plurality of pieces of hardware are configured to execute one or a plurality of processes of a processor, the plurality of pieces of hardware may be present in apparatuses physically separated from each other, or may be present in the same apparatus. In addition, in any of the embodiments, the order of each process by the processor 33 is not limited to the above order, and may be appropriately changed. In addition, hardware is implemented in a form of an electric circuit (circuitry) in which circuit elements, such as semiconductor elements, are combined.

Further, the program may be software, such as firmware or microcode. In addition, the program may be, for example, a program module group, and each function thereof may be realized by a processor configured to execute each function. The program may be a program code or a plurality of code segments stored in one or a plurality of non-transitory computer-readable media (for example, storage media or other storage). The program may be divided and stored in a plurality of non-transitory computer-readable media present in apparatuses physically separated from each other. The program code or the code segment may represent any combination of procedures, functions, subprograms, routines, subroutines, modules, software packages, classes, instructions, data structures, or program statements. The program code or code segments may be connected to other code segments or to hardware circuits by transmitting and receiving information, data, arguments, parameters, or contents of memory.

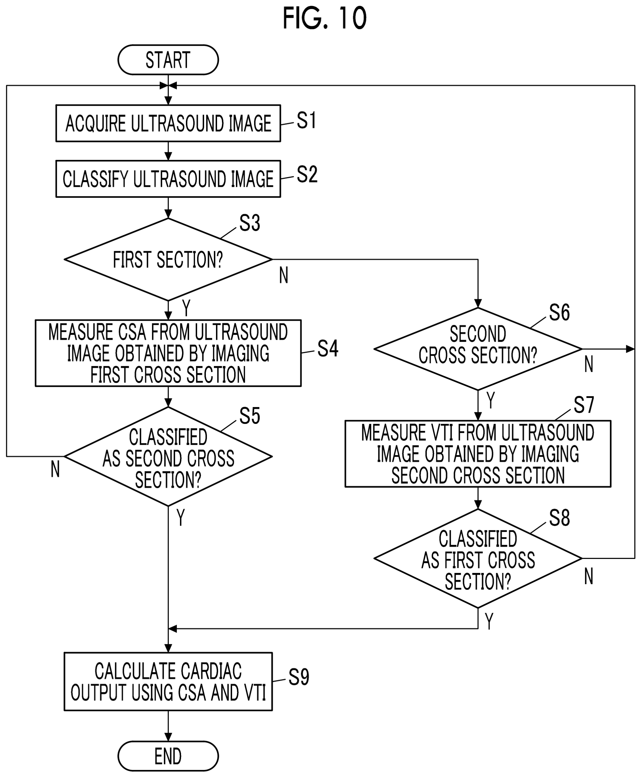

Hereinafter, an operation of the ultrasound diagnostic apparatus according to Embodiment 1 will be described with reference to a flowchart shown in FIG. 10. Here, an example will be described in which an ultrasound image representing a parasternal left ventricular long axis view is acquired as the ultrasound image U1 representing the first cross section, and an ultrasound image representing an apical five-chamber view is acquired as the ultrasound image U2 representing the second cross section.

In step S1, the user disposes the ultrasound probe 1 on the body surface of the subject, and the image acquisition unit 32 acquires ultrasound images of a plurality of frames. In this case, under the control of the apparatus control unit 30, the transmission and reception of the ultrasound wave from the plurality of transducers of the transducer array 11 are started in accordance with the drive signal from the pulser 41 of the transmission and reception circuit 12 of the ultrasound probe 1, the ultrasound echo from the subject is received by the plurality of transducers of the transducer array 11, and the reception signal as the analog signal is output to the amplification unit 42, is amplified, and then is subjected to the AD conversion via the AD conversion unit 43 to acquire the reception data.

The reception focus processing is performed on the reception data by the beam former 44, the sound ray signal generated by the reception focus processing is sent to the image generation unit 21 of the apparatus main body 2, and thus the ultrasound image is generated by the image generation unit 21. In this case, the signal processing unit 45 of the image generation unit 21 performs the correction of the attenuation in accordance with the depth of the reflection position of the ultrasound wave and the envelope detection processing on the sound ray signal, the DSC 46 performs the conversion into the image signal in accordance with the normal television signal scanning method, and the image processing unit 47 performs various types of necessary image processing such as gradation processing.

In step S2, the image classification unit 25 classifies whether the ultrasound images U1 and U2 of the plurality of frames acquired in step S1 are the ultrasound image U1 obtained by imaging the first cross section or the ultrasound image U2 obtained by imaging the second cross section. The image classification unit 25 can classify the ultrasound images U1 and U2 of the plurality of frames by, for example, a template matching method or a method using a trained model by machine learning.

In step S3, the apparatus control unit 30 determines whether or not the ultrasound images of the plurality of frames acquired in step S1 are classified into the ultrasound image U1 obtained by imaging the first cross section in step S2. In step S3, in a case where it is determined that the ultrasound images of the plurality of frames are classified into the ultrasound image U1 obtained by imaging the first cross section, the processing proceeds to step S4.

In step S4, the first measurement unit 26 measures the cross-sectional area of the left ventricular outflow tract T based on the ultrasound image U1 obtained by imaging the first cross section classified in step S2. In the ultrasound image U1 of the plurality of frames, as shown in FIG. 7, the first measurement unit 26 disposes a measurement line ML1 on the left ventricular outflow tract T and measures the length of the disposed measurement line ML1 as the left ventricular outflow tract diameter. In this case, the first measurement unit 26 can detect the aortic valve by, for example, a method using template matching or machine learning, and can automatically dispose the measurement line ML1 at a position within a predetermined range such as 1 cm to 2 cm from the detected aortic valve toward the left ventricle. The first measurement unit 26 detects, for example, a frame in which an angle between a pair of aortic valve leaflets is maximized as a mid-systolic frame, and calculates the cross-sectional area of the left ventricular outflow tract T based on the left ventricular outflow tract diameter in the mid-systolic frame.

In step S5, the apparatus control unit 30 determines whether or not the ultrasound images of the plurality of frames acquired in step S1 are already classified as the ultrasound image U2 obtained by imaging the second cross section in step S2. In step S5, in a case where it is determined that the ultrasound images of the plurality of frames are not already classified as the ultrasound image U2 obtained by imaging the second cross section, the processing returns to step S1 to acquire the ultrasound image U2 obtained by imaging the second cross section, and the ultrasound images of the plurality of frames are acquired again. In this case, the user adjusts the position and the posture of the ultrasound probe 1 such that the ultrasound image U2 representing the second cross section can be acquired. In subsequent step S2, the ultrasound images of the plurality of frames are classified by the image classification unit 25.

In step S3, in a case where the ultrasound images of the plurality of frames acquired in step S1 are not classified into the ultrasound image U1 obtained by imaging the first cross section, the processing proceeds to step S6.

In step S6, the apparatus control unit 30 determines whether or not the ultrasound images of the plurality of frames acquired in step S1 are classified into the ultrasound image U2 obtained by imaging the second cross section in step S2. In step S6, in a case where it is determined that the ultrasound images of the plurality of frames are not classified into the ultrasound image U2 obtained by imaging the second cross section, the ultrasound images of the plurality of frames do not correspond to the first cross section or the second cross section, and thus the processing returns to step S1 to acquire the ultrasound images of the plurality of frames again. Thereafter, the processing in step S2 and subsequent steps is performed. In step S6, in a case where it is determined that the ultrasound images of the plurality of frames are classified into the ultrasound image U2 obtained by imaging the second cross section, the processing proceeds to step S7.

In step S7, the second measurement unit 27 calculates the velocity-time integral of the left ventricular outflow tract blood flow based on the ultrasound image U2 obtained by imaging the second cross section classified in step S2. In this case, the second measurement unit 27 recognizes the systole of the heart based on, for example, any one of the closure of the mitral valve, the presence or absence of depiction of the aortic valve, the area of the left ventricle, or the depth of at least one set reference point in the ultrasound images U2 of the plurality of frames, and sets the Doppler gate G on the left ventricular outflow tract T in the ultrasound image U2 of the frame corresponding to the systole as shown in FIG. 9. The second measurement unit 27 can detect, for example, the left ventricular outflow tract T in the ultrasound image U2 of the frame corresponding to the systole, and can automatically set the Doppler gate G on the detected left ventricular outflow tract T. The second measurement unit 27 further acquires the Doppler waveform in the Doppler gate G, and calculates the velocity-time integral based on the Doppler waveform.

In step S8, the apparatus control unit 30 determines whether or not the ultrasound images of the plurality of frames acquired in step S1 are already classified as the ultrasound image U1 obtained by imaging the first cross section in step S2. In step S8, in a case where it is determined that the ultrasound images of the plurality of frames are not already classified as the ultrasound image U1 obtained by imaging the first cross section, the processing returns to step S1 to acquire the ultrasound image U1 obtained by imaging the first cross section, and the ultrasound images of the plurality of frames are acquired again. In this case, the user adjusts the position and the posture of the ultrasound probe 1 such that the ultrasound image U1 representing the first cross section can be acquired. In subsequent step S2, the ultrasound images of the plurality of frames are classified by the image classification unit 25.

In a case where it is determined in step S5 that the ultrasound images of the plurality of frames are already classified as the ultrasound image U2 obtained by imaging the second cross section, or in a case where it is determined in step S8 that the ultrasound images of the plurality of frames are already classified as the ultrasound image U1 obtained by imaging the first cross section, the processing proceeds to step S9.

In step S9, the cardiac output calculation unit 28 calculates the cardiac output using the cross-sectional area of the left ventricular outflow tract T measured in step S4 and the velocity-time integral of the left ventricular outflow tract blood flow measured in step S7. In this case, the cardiac output calculation unit 28 calculates a so-called stroke volume by multiplying the cross-sectional area of the left ventricular outflow tract T by the velocity-time integral, and calculates the cardiac output by multiplying the calculated stroke volume by the heart rate.

All of the processing of steps S1 to S9 is automatically performed in a case where the user disposes the ultrasound probe 1 on the body surface of the subject. Therefore, the user does not need to perform a complicated operation to measure the cardiac output, and can easily and quickly measure the cardiac output.

In a case where the processing of step S9 is completed in this manner, the operation of the ultrasound diagnostic apparatus according to the flowchart of FIG. 10 is completed.

As described above, with the ultrasound diagnostic apparatus according to Embodiment 1 of the present invention, the image classification unit 25 classifies whether the input image is the ultrasound image obtained by imaging the first cross section or the second cross section, the first measurement unit 26 measures the cross-sectional area of the left ventricular outflow tract T based on the input image in a case where the input image is classified as the ultrasound image U1 obtained by imaging the first cross section, the second measurement unit 27 measures the velocity-time integral of the left ventricular outflow tract blood flow based on the input image in a case where the input image is classified as the ultrasound image U2 obtained by imaging the second cross section, and the cardiac output calculation unit 28 calculates the cardiac output using the cross-sectional area measured by the first measurement unit 26 and the velocity-time integral measured by the second measurement unit 27. Therefore, the cardiac output can be easily and quickly calculated.

A case has been described in which the transmission and reception circuit 12 is provided in the ultrasound probe 1, but the transmission and reception circuit 12 may be provided in the apparatus main body 2.

Further, a case has been described in which the image generation unit 21 is provided in the apparatus main body 2, but the image generation unit 21 may be provided in the ultrasound probe 1.

The apparatus main body 2 may be a so-called stationary type, a portable type that is easy to carry, or a so-called handheld type that is configured by, for example, a smartphone or a tablet type computer. As described above, the type of the device that constitutes the apparatus main body 2 is not particularly limited.

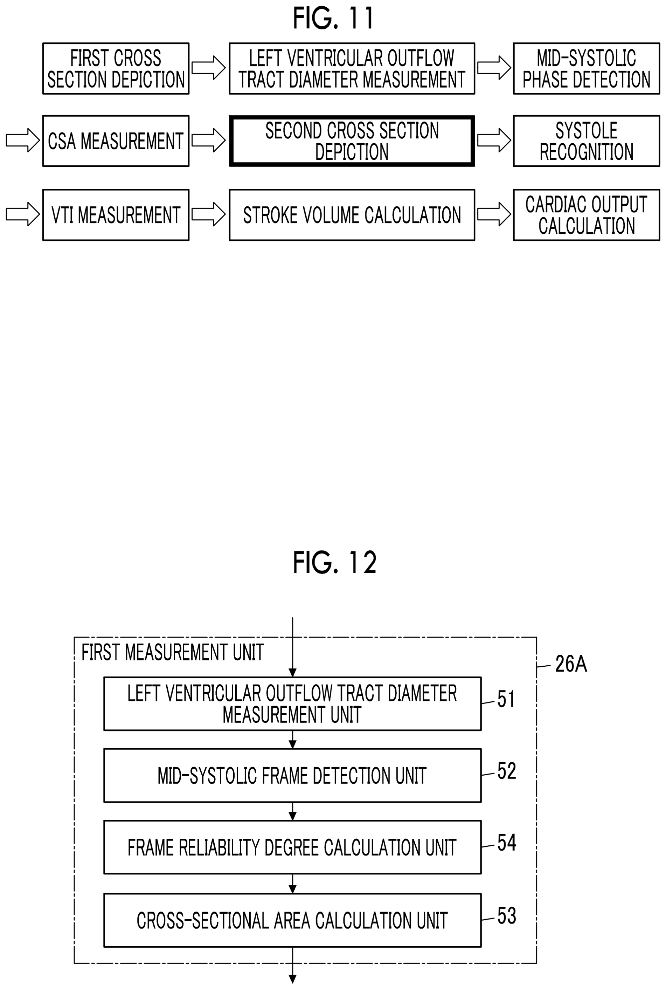

The display control unit 22 can display a series of processing steps, for example, as shown in FIG. 11 on the monitor 23 while the ultrasound diagnostic apparatus performs a series of processing from the acquisition of the ultrasound images of the plurality of frames to the calculation of the cardiac output. In this case, the display control unit 22 can display the processing step currently being executed among the series of processing steps in an emphasized manner. In the example of FIG. 11, an example is shown in which the frame line of the processing step of "second cross section depiction" is displayed in a display aspect different from the frame lines of the other processing steps. The display control unit 22 can display the processing step in an emphasized manner, for example, by changing the color of the display of the processing step, the display aspect of the frame line, or the transparency with respect to the display of the other processing steps.

The left ventricular outflow tract diameter can be accurately measured in a case where the position and the posture of the ultrasound probe 1 are stable in time series. Therefore, the first measurement unit 26 can perform the measurement, for example, only in a case where the position and the posture of the ultrasound probe 1 are stable. In this case, the apparatus main body 2 can comprise a first measurement unit 26A shown in FIG. 12. In the first measurement unit 26A, a frame reliability degree calculation unit 54 is further inserted between the mid-systolic frame detection unit 52 and the cross-sectional area calculation unit 53 in the first measurement unit 26 shown in FIG. 6.

The frame reliability degree calculation unit 54 calculates the reliability degree of the frame based on the certainty of the classification in the image classification unit 25 or based on the time-series change of the left ventricular outflow tract diameter measured by the left ventricular outflow tract diameter measurement unit 51.

In a case where the image classification unit 25 classifies the plurality of ultrasound images, the image classification unit 25 can calculate the certainty of the classification, that is, the likelihood of the ultrasound images of the plurality of frames being the ultrasound image U1 corresponding to the first cross section and the likelihood of the ultrasound images of the plurality of frames being the ultrasound image U2 corresponding to the second cross section. The image classification unit 25 can calculate, for example, the similarity between the typical image pattern representing the ultrasound image U1 corresponding to the first cross section and each of the ultrasound images of the plurality of frames, and the similarity between the typical image pattern representing the ultrasound image U2 corresponding to the second cross section and each of the ultrasound images of the plurality of frames, as the certainty of the classification. In addition, the image classification unit 25 can calculate so-called intermediate data calculated in a case where a trained model in machine learning classifies the ultrasound image, as the certainty of the classification.

The frame reliability degree calculation unit 54 can calculate the certainty of the classification in the image classification unit 25 as the reliability degree of the frame. The first measurement unit 26 can perform the measurement only on the frame in which the reliability degree of the frame is equal to or greater than a predetermined reliability degree threshold value.

In addition, the left ventricular outflow tract diameter measured by the left ventricular outflow tract diameter measurement unit 51 is periodically changed in accordance with the heartbeat in a case where the position and the posture of the ultrasound probe 1 are fixed. Therefore, the frame reliability degree calculation unit 54 can calculate, for example, the reliability degree of the frame in which the left ventricular outflow tract diameter is periodically changed in the time series as 1, and the reliability degree of the frame in which the left ventricular outflow tract diameter is not periodically changed in the time series as 0. In this case, the first measurement unit 26 can perform the measurement only on the frame in which the reliability degree of the frame is 1, by setting the predetermined reliability degree threshold value to 1.

The display control unit 22 can display the reliability degree of the frame calculated by the frame reliability degree calculation unit 54 on the monitor 23 as a numerical value. In addition, in a case where the frame reliability degree calculation unit 54 calculates the intermediate data in the machine learning as the reliability degree, the reliability degree can be calculated for each pixel in the ultrasound image. In this case, the display control unit 22 can display the reliability degree calculated for each pixel in the ultrasound image as a so-called heat map in a superimposed manner on the ultrasound image. The user can check the displayed reliability degree to understand that the examination is being appropriately performed, and can proceed with the examination. In addition, in a case where the user determines that the reliability degree is low, the user can perform, for example, adjustment of the position or the posture of the ultrasound probe 1, manual adjustment of the measurement line ML1 via the input device 31, or the like.

It has been described that the mid-systolic frame detection unit 52 of the first measurement unit 26 detects the ultrasound image U1 of the frame in which the angle between the pair of aortic valve leaflets is the maximum as the ultrasound image U1 of the mid-systolic frame, but the aortic valve may not be detected for some reason, such as the ultrasound image U1 being unclear due to a change in the position or the posture of the ultrasound probe 1, and as a result, the mid-systolic frame may not be detected. In this case, the mid-systolic frame detection unit 52 can select, for example, the frame in which the maximum left ventricular outflow tract diameter among the plurality of left ventricular outflow tract diameters measured for the ultrasound images U1 of the plurality of frames by the left ventricular outflow tract diameter measurement unit 51 is measured, as the frame used by the cross-sectional area calculation unit 53 in a case of calculating the cross-sectional area of the left ventricular outflow tract T.

In addition, in a case where the mid-systolic frame cannot be detected, the mid-systolic frame detection unit 52 can also detect, for example, a frame representing the end-diastolic phase of the heart, and select the frame representing the end-diastolic phase as the frame used by the cross-sectional area calculation unit 53 in a case of calculating the cross-sectional area of the left ventricular outflow tract T. The mid-systolic frame detection unit 52 can recognize the left ventricular region in the ultrasound images U1 of the plurality of frames by, for example, a method using template matching or a trained model in machine learning, calculate the area of the recognized left ventricular region, and detect the frame in the phase in which the area of the left ventricular region is the maximum in the time series as the frame representing the end-diastolic phase. In a case where the mid-systolic frame cannot be detected, the mid-systolic frame detection unit 52 can also allow the user to select, for example, which of the frame in which the maximum left ventricular outflow tract diameter is measured or the frame representing the end-diastolic phase is to be used by the cross-sectional area calculation unit 53 in a case of calculating the cross-sectional area of the left ventricular outflow tract T, via the input device 31 by displaying a message on the monitor 23.

In addition, in general, due to the anatomical structure of the heart, it may be easier to depict the ultrasound image representing the parasternal left ventricular long axis view than to depict the ultrasound image representing the apical five-chamber view. In addition, it may be more accurate to measure the left ventricular outflow tract diameter by measuring the left ventricular outflow tract diameter using the ultrasound image representing the parasternal left ventricular long axis view than by measuring the left ventricular outflow tract diameter using the ultrasound image representing the apical five-chamber view. Therefore, the apparatus control unit 30 can control each unit of the apparatus main body 2 such that, for example, the left ventricular outflow tract diameter is measured using the ultrasound image representing the parasternal left ventricular long axis view, and then the velocity-time integral is measured using the ultrasound image representing the apical five-chamber view.

In addition, the display control unit 22 can display, for example, a message prompting the user to capture the parasternal left ventricular long axis view, such as "Please capture the parasternal left ventricular long axis view", on the monitor 23 until the ultrasound image representing the parasternal left ventricular long axis view is classified by the image classification unit 25, and can display, for example, a message prompting the user to capture the apical five-chamber view, such as "Please capture the apical five-chamber view", on the monitor 23 after the ultrasound image representing the parasternal left ventricular long axis view is classified by the image classification unit 25.

In addition, in a medical field, in a case where the user, such as a doctor, checks the ultrasound image representing the parasternal left ventricular long axis view as the first cross section and determines that there is no abnormality in the heart function of the subject, the user may omit the depiction of the second cross section. Therefore, the apparatus control unit 30 can also stop, for example, a series of processing for calculating the stroke volume or the cardiac output based on an instruction from the user via the input device 31.

### Embodiment 2

The measurement values obtained by the first measurement unit 26 and the second measurement unit 27 may deviate from the normal values for some reason, such as a change in the position or posture of the ultrasound probe 1. In such a case, the ultrasound diagnostic apparatus can detect an error in the measurement value.

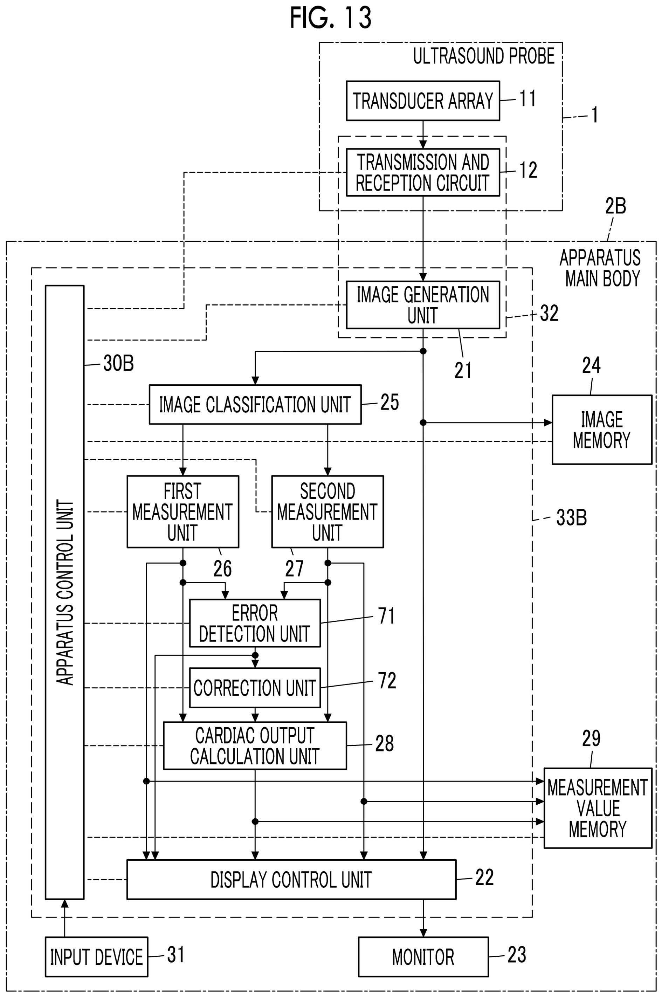

FIG. 13 shows a configuration of an ultrasound diagnostic apparatus according to Embodiment 2. The ultrasound diagnostic apparatus according to Embodiment 2 comprises an apparatus main body 2B instead of the apparatus main body 2, in the ultrasound diagnostic apparatus according to Embodiment 1 shown in FIG. 1. The apparatus main body 2B further comprises an error detection unit 71 and a correction unit 72, and comprises an apparatus control unit 30B instead of the apparatus control unit 30, in the apparatus main body 2 according to Embodiment 1. In the apparatus main body 2B, the error detection unit 71 is connected to the first measurement unit 26 and the second measurement unit 27. The correction unit 72, the display control unit 22, and the apparatus control unit 30B are connected to the error detection unit 71. The correction unit 72 is connected to the cardiac output calculation unit 28 and the apparatus control unit 30B. In addition, a processor 33B for the apparatus main body 2B is configured by the image generation unit 21, the display control unit 22, the image classification unit 25, the first measurement unit 26, the second measurement unit 27, the cardiac output calculation unit 28, the apparatus control unit 30B, the error detection unit 71, and the correction unit 72.

The error detection unit 71 detects an error related to the measurement value obtained by each of the first measurement unit 26 and the second measurement unit 27.

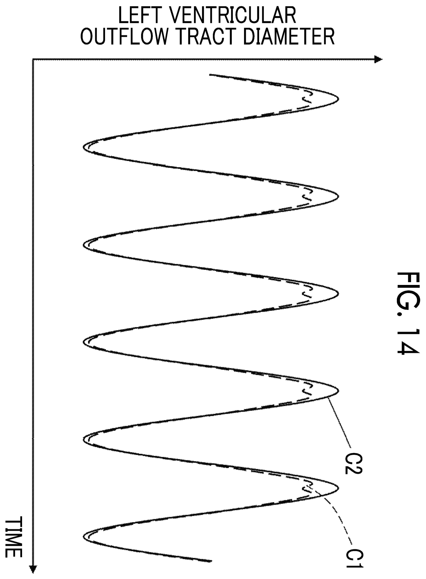

The error detection unit 71 detects, for example, an error in the left ventricular outflow tract diameter measured by the left ventricular outflow tract diameter measurement unit 51 of the first measurement unit 26. Ideally, the left ventricular outflow tract diameter changes periodically and regularly with the heartbeat, but the change in the measurement value may be irregular due to a factor such as a change in the position or the posture of the ultrasound probe 1 during the measurement. Therefore, as shown in FIG. 14, the error detection unit 71 can detect an error in the left ventricular outflow tract diameter by generating a time-series change curve C1 of the left ventricular outflow tract diameter measured by the left ventricular outflow tract diameter measurement unit 51 and comparing the generated time-series change curve C1 with a predetermined curve C2, which is, for example, a so-called B-spline curve. In a case where there is a portion where the time-series change curve C1 and the predetermined curve C2 do not match each other, such as a peak portion of the predetermined curve C2 in FIG. 14, the error detection unit 71 can determine that there is an error in the left ventricular outflow tract diameter.

In addition, it is generally known that an ideal value of the left ventricular outflow tract diameter is 2 cm. Therefore, the error detection unit 71 can also detect an error in the left ventricular outflow tract diameter by, for example, comparing the left ventricular outflow tract diameter measured by the left ventricular outflow tract diameter measurement unit 51 with a predetermined set value. As a specific example, the error detection unit 71 can compare the left ventricular outflow tract diameter measured by the left ventricular outflow tract diameter measurement unit 51 with a predetermined measurement value range including 2 cm, and determine that there is an error in the left ventricular outflow tract diameter in a case where the left ventricular outflow tract diameter is outside the determined measurement value range.

The error detection unit 71 can also detect, for example, an error in the velocity-time integral of the left ventricular outflow tract blood flow calculated by the velocity-time integral calculation unit 64 of the second measurement unit 27. Ideally, the Doppler waveform changes in a predetermined manner in time series, but the measurement value may not change as the ideal value due to a factor such as a change in the position or the posture of the ultrasound probe 1 during the measurement. In this case, a normal value may not be obtained as the velocity-time integral. Therefore, the error detection unit 71 can detect an error in the velocity-time integral by comparing the Doppler waveform acquired by the Doppler waveform acquisition unit 63 of the second measurement unit 27 with a predetermined curve, which is an ideal Doppler waveform of the left ventricular outflow tract blood flow. In a case where there is a portion where the Doppler waveform and the predetermined curve do not match each other, the error detection unit 71 can determine that there is an error in the velocity-time integral.

In addition, it is generally known that an ideal value of the velocity-time integral of the left ventricular outflow tract blood flow is 18 to 22 cm in the case of an adult. Therefore, the error detection unit 71 can also detect an error in the velocity-time integral by, for example, comparing the velocity-time integral calculated by the velocity-time integral calculation unit 64 with a predetermined set value. As a specific example, the error detection unit 71 can compare the velocity-time integral calculated by the velocity-time integral calculation unit 64 with, for example, a predetermined measurement value range of 18 to 22 cm, and determine that there is an error in the velocity-time integral in a case where the velocity-time integral is outside the predetermined measurement value range.

The correction unit 72 corrects the measurement value of the first measurement unit 26 and the measurement value of the second measurement unit 27 in which the error is detected by the error detection unit 71. The correction unit 72 can correct the left ventricular outflow tract diameter in which the error is detected by the error detection unit 71 based on the cross-sectional area of the left ventricular outflow tract T calculated by the cross-sectional area calculation unit 53 in the past. The correction unit 72 can, for example, input the ultrasound image U1 to a first trained model in machine learning in which a landmark such as an aortic valve or a mitral valve in the ultrasound image U1 in which the first cross section is imaged is learned in advance, to output a certainty of the landmark for each pixel as a heat map, and further input the heat map of the landmark and the cross-sectional area of the left ventricular outflow tract T calculated by the first measurement unit 26 to a second trained model in machine learning in which a relationship between the heat map of the landmark and the cross-sectional area of the left ventricular outflow tract T is learned in advance, to output the corrected cross-sectional area of the left ventricular outflow tract T.

In addition, the correction unit 72 can also correct the velocity-time integral in which the error is detected by the error detection unit 71 based on the velocity-time integral of the left ventricular outflow tract blood flow calculated by the velocity-time integral calculation unit 64 in the past. The correction unit 72 can, for example, input the ultrasound image U2 to a first trained model in machine learning in which a landmark such as an aortic valve or a mitral valve in the ultrasound image U2 in which the second cross section is imaged is learned in advance, to output a certainty of the landmark for each pixel as a heat map, and further input the heat map of the landmark, the velocity-time integral of the left ventricular outflow tract blood flow, and the Doppler waveform acquired by the second measurement unit 27 to a second trained model in machine learning in which a relationship between the heat map of the landmark, the velocity-time integral of the left ventricular outflow tract blood flow, and the Doppler waveform is learned in advance, to output the corrected velocity-time integral of the left ventricular outflow tract blood flow.

As described above, with the ultrasound diagnostic apparatus according to Embodiment 2, since the error in the measurement value is automatically corrected by the correction unit 72, the user can easily obtain an appropriate correction value.

In Embodiment 2, the description has been made in which the measurement value in which the error is detected by the error detection unit 71 is automatically corrected by the correction unit 72, but for example, the error can be displayed on the monitor 23 without correcting the error. In this case, the user can take measures such as adjusting the position and the posture of the ultrasound probe 1 to prevent the error from occurring in the measurement value, for example, by checking the error.

Explanation of References
1: ultrasound probe
2: apparatus main body
11: transducer array
12: transmission and reception circuit
21: image generation unit
22: display control unit
23: monitor
24: image memory
25: image classification unit
26, 26A: first measurement unit
27: second measurement unit
28: cardiac output calculation unit
29: measurement value memory
30, 30B: apparatus control unit
31: input device
32: image acquisition unit
33, 33B: processor
41: pulser
42: amplification unit
43: AD conversion unit
44: beam former
45: signal processing unit
46: DSC
47: image processing unit
51: left ventricular outflow tract diameter measurement unit
52: mid-systolic frame detection unit
53: cross-sectional area calculation unit
54: frame reliability degree calculation unit
61: systole recognition unit
62: gate setting unit
63: Doppler waveform acquisition unit
64: velocity-time integral calculation unit
71: error detection unit
72: correction unit
C1: time-series change curve
C2: predetermined curve
ML1: measurement line
G: Doppler gate
T: left ventricular outflow tract
U1, U2: ultrasound image

## Claims

1. An ultrasound diagnostic apparatus that measures cardiac output based on ultrasound images of a plurality of frames obtained by respectively imaging a first cross section and a second cross section, which are different from each other, of a heart of a subject, the ultrasound diagnostic apparatus comprising:
an image classification unit (25) that classifies whether an input image is an ultrasound image obtained by imaging the first cross section or the second cross section;
a first measurement unit (26, 26A) that measures a cross-sectional area of a left ventricular outflow tract based on the input image in a case where the image classification unit (25) classifies the input image as the ultrasound image obtained by imaging the first cross section;
a second measurement unit (27) that measures a velocity-time integral of a left ventricular outflow tract blood flow based on the input image in a case where the image classification unit (25) classifies the input image as the ultrasound image obtained by imaging the second cross section; and
a cardiac output calculation unit (28) that calculates the cardiac output by using the cross-sectional area measured by the first measurement unit (26, 26A) and the velocity-time integral measured by the second measurement unit (27).

2. The ultrasound diagnostic apparatus according to claim 1,
wherein the image classification unit (25) classifies the input image using a parasternal left ventricular long axis view as the first cross section and an apical five-chamber view or an apical three-chamber view as the second cross section.

3. The ultrasound diagnostic apparatus according to either claim 1 or claim 2,
wherein the first measurement unit (26, 26A) includes
a left ventricular outflow tract diameter measurement unit (51) that measures a left ventricular outflow tract diameter based on the input image,
a mid-systolic frame detection unit (52) that detects a mid-systolic frame based on the input image, and
a cross-sectional area calculation unit (53) that calculates the cross-sectional area of the left ventricular outflow tract from the left ventricular outflow tract diameter measured by the left ventricular outflow tract diameter measurement unit (51) in the mid-systolic frame detected by the mid-systolic frame detection unit (52).

4. The ultrasound diagnostic apparatus according to claim 3,
wherein the left ventricular outflow tract diameter measurement unit (51) measures the left ventricular outflow tract diameter at a position within a predetermined distance from an insertion point of an aortic valve toward a heart side.

5. The ultrasound diagnostic apparatus according to claim 3 or 4,
wherein the mid-systolic frame detection unit (52) detects a frame in which an angle between a pair of aortic valve leaflets is maximized as the mid-systolic frame.

6. The ultrasound diagnostic apparatus according to any one of claims 3 to 5,
wherein the first measurement unit (26A) includes a frame reliability degree calculation unit (54) that calculates a reliability degree of the frame, and
the left ventricular outflow tract diameter measurement unit (51) measures the left ventricular outflow tract diameter based on the frame in which the reliability degree calculated by the frame reliability degree calculation unit (54) is equal to or greater than a predetermined reliability degree threshold value.

7. The ultrasound diagnostic apparatus according to claim 6,
wherein the frame reliability degree calculation unit (54) calculates the reliability degree based on a certainty of classification in the image classification unit or based on a time-series change in the left ventricular outflow tract diameter measured by the left ventricular outflow tract diameter measurement unit (51).

8. The ultrasound diagnostic apparatus according to any one of claims 1 to 7, in particular to claim 2,
wherein the second measurement unit (27) includes
a systole recognition unit (61) that recognizes a systole based on the input image,
a gate setting unit (62) that sets a Doppler gate in the input image recognized as the systole by the systole recognition unit (61),
a Doppler waveform acquisition unit (63) that acquires a Doppler waveform in the Doppler gate set by the gate setting unit (62), and
a velocity-time integral calculation unit (64) that calculates the velocity-time integral based on the Doppler waveform acquired by the Doppler waveform acquisition unit (63).

9. The ultrasound diagnostic apparatus according to claim 8,
wherein the systole recognition unit (61) recognizes the systole based on any one of closure of a mitral valve, presence or absence of depiction of an aortic valve, an area of a left ventricle, or a depth of a reference point set in the input image.

10. The ultrasound diagnostic apparatus according to claim 8 or 9,
wherein the first measurement unit (26, 26A) includes a left ventricular outflow tract diameter measurement unit (51) that measures a left ventricular outflow tract diameter based on the input image, and
the gate setting unit (62) sets the Doppler gate in the input image based on the left ventricular outflow tract diameter measured by the left ventricular outflow tract diameter measurement unit (51) in the first measurement unit (26, 26A).

11. The ultrasound diagnostic apparatus according to any one of claims 1 to 10, in particular to claim 3,
wherein the cardiac output calculation unit (28)
calculates a heart rate based on the number of plots of the left ventricular outflow tract diameter by the left ventricular outflow tract diameter measurement unit (51) between two consecutive mid-systolic frames detected by the mid-systolic frame detection unit (52), and
calculates the cardiac output from the cross-sectional area measured by the first measurement unit (26, 26A), the velocity-time integral measured by the second measurement unit, and the calculated heart rate.

12. The ultrasound diagnostic apparatus according to any one of claims 1 to 11, in particular to claim 3, further comprising:
an error detection unit (71) that detects an error in the left ventricular outflow tract diameter measured by the left ventricular outflow tract diameter measurement unit (51).

13. The ultrasound diagnostic apparatus according to claim 12,
wherein the error detection unit (71) detects the error in the left ventricular outflow tract diameter by comparing a time-series change curve of the left ventricular outflow tract diameter measured by the left ventricular outflow tract diameter measurement unit (51) with a predetermined curve or by comparing the left ventricular outflow tract diameter measured by the left ventricular outflow tract diameter measurement unit (51) with a predetermined set value.

14. The ultrasound diagnostic apparatus according to either claim 12 or claim 13, further comprising:
a correction unit (72) that corrects the left ventricular outflow tract diameter in which the error is detected by the error detection unit (71) based on the cross-sectional area of the left ventricular outflow tract calculated by the cross-sectional area calculation unit (53) in the past.

15. The ultrasound diagnostic apparatus according to any one of claims 1 to 14, in particular to claim 8, further comprising:
an error detection unit (71) that detects an error in the velocity-time integral calculated by the velocity-time integral calculation unit (64).

16. The ultrasound diagnostic apparatus according to claim 15,
wherein the error detection unit (71) detects the error in the velocity-time integral by comparing a Doppler curve obtained by tracing the Doppler waveform acquired by the Doppler waveform acquisition unit (63) with a predetermined curve or by comparing the velocity-time integral calculated by the velocity-time integral calculation unit (64) with a predetermined set value.

17. The ultrasound diagnostic apparatus according to either claim 15 or claim 16, further comprising:
a correction unit (72) that corrects the velocity-time integral in which the error is detected by the error detection unit (71) based on the velocity-time integral calculated by the velocity-time integral calculation unit (64) in the past.

18. A control method for an ultrasound diagnostic apparatus that measures cardiac output based on an ultrasound image of a plurality of frames obtained by respectively imaging a first cross section and a second cross section, which are different from each other, of a heart of a subject, the control method comprising:
classifying whether an input image is an ultrasound image obtained by imaging the first cross section or the second cross section;
measuring a cross-sectional area of a left ventricular outflow tract based on the input image classified as the ultrasound image obtained by imaging the first cross section;
measuring a velocity-time integral of a left ventricular outflow tract blood flow based on the input image classified as the ultrasound image obtained by imaging the second cross section; and
calculating the cardiac output using the measured cross-sectional area and the measured velocity-time integral.
